(19) Europäisches Patentamt European Patent Office Office européen des brevets

(11) **EP 2 257 217 B1**

(12) **EUROPEAN PATENT SPECIFICATION**

(45) Date of publication and mention of the grant of the patent:
**12.04.2017 Bulletin 2017/15**

(21) Application number: **09713929.9**

(22) Date of filing: **02.02.2009**

(51) Int Cl.:
*A61B 5/021* *(2006.01)*     *A61B 5/024* *(2006.01)*

(86) International application number:
**PCT/IB2009/050416**

(87) International publication number:
**WO 2009/107007 (03.09.2009 Gazette 2009/36)**

(54) **HEMODYNAMIC MONITORS AND ALARMS**

HÄMODYNAMISCHE MONITORE UND ALARME

MONITEURS HÉMODYNAMIQUES ET ALARMES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **27.02.2008 US 31708**

(43) Date of publication of application:
**08.12.2010 Bulletin 2010/49**

(73) Proprietor: **Koninklijke Philips N.V.**
**5656 AE Eindhoven (NL)**

(72) Inventor: **ZONG, Wei**
**Cleveland**
**Ohio 44143 (US)**

(74) Representative: **van Velzen, Maaike Mathilde**
**Philips Intellectual Property & Standards**
**High Tech Campus 5**
**5656 AE Eindhoven (NL)**

(56) References cited:
**WO-A-2007/060559     US-A- 5 743 268**

- BECKER K ET AL: "Fuzzy logic approaches to intelligent alarms" IEEE ENGINEERING IN MEDICINE AND BIOLOGY MAGAZINE 1994 NOV-DEC IEEE US, vol. 13, no. 5, November 1994 (1994-11), pages 710-716, XP002525255
- KIZAKEVICH PAUL N ET AL: "Beat-by-beat monitoring of systemic vascular resistance during head-up tilt for assessment of orthostatic stress response" PROCEEDINGS OF THE IEEE SYMPOSIUM ON COMPUTER-BASED MEDICAL SYSTEMS 1994 IEEE, 1994, pages 82-87, XP002525256
- HARRISON MJ: "Effect of age on physiological variables during anaesthesia" AUSTRALIAN AND NEW ZEALAND COLLEGE OF ANAESTHETISTS ANNUAL SCIENTIFIC MEETING, [Online] 2006, XP002525257 ADELAIDE, AUSTRALIA Retrieved from the Internet: URL:http://www.anzca.edu.au/events/asm/asm 2006/harrison_3.htm> [retrieved on 2009-04-24]

**Description**

The following relates to the medical arts. It finds application in medical monitoring, medical alarm systems, and the like, for use in hospitals, urgent care centers, nursing homes, assisted care facilities, home medical monitoring, and the like.

Vasoconstriction medications, also called "vasopressors" cause vascular constriction and consequent increase in the arterial blood pressure (ABP). Accordingly, administration of a vasopressor is a recommended remedial action when a patient is in an abnormal hemodynamic state due to vascular resistance problems. Timely detection or prediction of the need for vasopressor intervention is crucial, because the time frame for intervention is relatively short and vital organs such as the brain can suffer irreversible damage leading to permanent debility or death if the vasopressor intervention is delayed.

To assess the desirability of vasopressor intervention, it is known that one should measure the mean aortic pressure (MAP), the cardiac output (CO), and the central venous pressure (CVP). These three quantities enable determination of the systemic vascular resistance (SVR) according to the relation MAP=(CO×SVR)+CVP and vasopressor intervention is indicated if the SVR falls below a threshold value.

In practice, measurement of central venous pressure is very invasive, so this quantity is generally assumed to have a negligible effect on the SVR. Measurement of cardiac output is also highly invasive, and CO measurements do not provide a continuously generated CO value that can be conveniently monitored. Unfortunately, CO is usually not negligible in determining SVR.

Measurement of the mean aortic pressure (MAP) is also very invasive. However, the arterial blood pressure (ABP) is readily measured in a non-invasive or minimally invasive manner, for example using a sphygmometer or an arterial line. The mean value of the ABP also approximately equals the MAP. In view of the invasiveness of the MAP, CO, and CVP measurements, it is commonplace for only ABP to be measured.

As a consequence, medical personnel are left to make the determination as to whether a vasopressor should be administered based on incomplete information. Under these circumstances, different physicians can make different qualitative judgments in deciding if and when the need for vasopressor intervention arises, typically relying on the available ABP measurements, other measured physiological parameters, and other information such as the patient's overall appearance, pre-existing medical conditions, or so forth. This situation leads to a lack of uniformity in medical care and can result in unnecessary vasopressor administration, or conversely failure to administer a vasopressor that would have been medically beneficial.

WO2007/060559 A2 discloses a medical monitoring system in which one or more biometric monitors collect data of a plurality of monitored biometric parameters. An expression evaluator evaluates a user-defined expression incorporating one or more of the monitored biometric parameters to generate data for a user-defined biometric parameter. A further processing component performs processing and display of data of at least one parameter selected from the monitored biometric parameters and the user-defined biometric parameter. D1 further discloses a user-defined function which represents an estimate of the systemic vascular resistance (SVR) as a function of a mean arterial blood pressure ("MABP") library function selected from a library functions list, the central venous pressure library ("CVP") function selected from the library functions list, represents the mean pulmonary artery pressure ("MPAP") library function selected from the library functions list, and the left-atrial pressure ("LAP") library function selected from the library functions list.

BECKER K ET AL: "Fuzzy logic approaches to intelligent alarms" IEEE ENGINEERING IN MEDICINE AND BIOLOGY MAGAZINE 1994 NOV-DEC IEEE US, vol. 13, no. 5, November 1994 (1994-11), pages 710-716, XP002525255 discloses the use of fuzzy logic in medical monitoring. As an example a hemodynamic state variable model is transformed into a fuzzy system. Variables that are applied are arterial systolic pressure (AP) and left atrial pressure (LAP).

The following provides a new and improved apparatuses and methods which overcome the above-referenced problems and others.

In accordance with one aspect, a hemodynamic monitoring instrument is disclosed, comprising a processor arranged to receive a physiological parameter indicative of heart rate and a physiological parameter indicative of arterial blood pressure and configured to compute a hemodynamic parameter correlating with systemic vascular resistance (SVR) based on the received physiological parameter indicative of heart rate and the received physiological parameter indicative of arterial blood pressure; and an output device including least one of (i) a display configured to display the computed hemodynamic parameter and (ii) an alarm configured to generate a perceptible signal responsive to the computed hemodynamic parameter satisfying an alarm criterion, wherein the processor is configured to compute the hemodynamic parameter correlating with SVR based on:a first fuzzy membership function indicative of whether a heart rate indicated by the physiological parameter indicative of heart rate is slightly high or high;a second fuzzy membership function indicative of whether an arterial blood pressure indicated by the physiological parameter indicative of arterial blood pressure is low; and a third fuzzy membership function indicative of whether the arterial blood pressure indicated by the physiological parameter indicative of arterial blood pressure is very low,wherein the processor is further configured to compute the hemodynamic parameter substantially correlating with SVR based on: a first term defined by conjunctive combination of the first fuzzy membership function and the second fuzzy membership function, and a second term corresponding to the third fuzzy membership function,the hemodynamic parameter quantifying the heuristic "ABP is low AND HR is slightly high or high" OR "ABP is very low" where APB denotes an arterial blood pressure indicated by the physiological parameter indicative of arterial blood pressure and HR denotes a heart rate indicated by the physiological parameter indicative of heart rate. In accordance with another aspect, a hemodynamic monitoring method is disclosed, comprising: computing a quantitative hemodynamic parameter that is (i) functionally dependent upon a quantitative heart rate (HR) measure and a quantitative arterial blood pressure (ABP) measure and (ii) correlates with systemic vascular resistance (SVR) and (iii) quantifies the heuristic "quantitative ABP measure is low AND quantitative HR measure is slightly high or high" OR " quantitative ABP measure is very low", wherein the computing of the quantitative hemodynamic parameter comprises:computing a first fuzzy variable indicative of whether the quantitative HR measure is slightly high or high;computing a second fuzzy variable indicative of whether the quantitative ABP measure is low; computing a third fuzzy variable indicative of whether the quantitative ABP measure is very low; computing a fuzzy composite variable combining the first, second, and third fuzzy variables using fuzzy combinational operators selected from the group consisting of fuzzy intersection and fuzzy union, the fuzzy composite variable correlating with systemic vascular resistance (SVR); and at least one of (i) displaying the quantitative hemodynamic parameter and(ii) generating a perceptible signal indicative of an abnormal hemodynamic condition conditional upon the computed hemodynamic parameter satisfying an alarm criterion. In accordance with certain other disclosed aspects, a computer medium is disclosed storing instructions executable to control a computer and display to perform the method of the immediately preceding paragraph, and a hemodynamic monitoring device is disclosed including a display and a processor programmed to perform the method of the immediately preceding paragraph.

One advantage resides in providing an instrument for determining on a quantitative basis when vasopressor intervention is indicated.

Another advantage resides in providing an instrument capable of making a timely determination as to when vasopressor intervention is indicated.

Another advantage resides in quantitatively monitoring a hemodynamic parameter closely related to systemic vascular resistance without resort to highly invasive measurement of the cardiac output.

Still further advantages of the present invention will be appreciated to those of ordinary skill in the art upon reading and understand the following detailed description.

FIGURE 1 diagrammatically shows a medical environment including a patient monitor including a hemodynamic monitoring instrument configured to indicate when vasopressor intervention is appropriate.

FIGURES 2 and 3 plot the shapes of the S-function and Z-function, respectively, used in the hemodynamic parameter computation implemented by the hemodynamic monitoring instrument of FIGURE 1.

FIGURES 4 and 5 diagrammatically show illustrative outputs of embodiments of the patient monitor of FIGURE 1 including the output of the hemodynamic monitoring instrument.

The hemodynamic monitors and alarms disclosed herein are based on some physiological insights. Starting with the relationship MAP=(CO×SVR)+CVP, if the central venous pressure (CVP) is assumed to be negligible, that is, CVP is approximated as zero, and mean arterial blood pressure (APB) is used as a substitute for the mean aortic pressure (MAP), then the systemic vascular resistance (SVR) can be approximated as SVR=ABP/CO. The cardiac output (CO) is equal to the cardiac stroke volume (SV) times the heart rate (HR), that is, CO=SV×HR. Physiologically, any variation in the cardiac output (CO) is typically due primarily to variation in the heart rate (HR), as the stroke volume (SV) is relatively constant for most patients and under most physiological conditions. Thus, CO∝HR captures the variability of the cardiac output (CO). Inserting this relationship into the expression for systemic vascular resistance (SVR) yields the relationship SVR∝ABP/HR. In other words, systemic vascular resistance (SVR) correlates with the hemodynamic parameter ABP/HR or with hemodynamic parameters proportional to or otherwise correlating with this ratio.

Vasopressor intervention is indicated if the systemic vascular resistance (SVR) is abnormally low, since the vasopressor intervention is intended to cause vascular constriction so as to increase the systemic vascular resistance (SVR). Based on the relationship SVR∝ABP/HR, one can predict that the systemic vascular resistance (SVR) will be low if the arterial blood pressure (APB) is low and the heart rate (HR) is high or slightly high. Since the heart rate (HR) cannot decrease too far (for example, HR does not fall below about 50 beats per minute for most people) one can also predict that the systemic vascular resistance (SVR) will be low if the arterial blood pressure (APB) is very low, regardless of the heart rate (HR) value.

On the other hand, if the heart rate (HR) is low, then the systemic vascular resistance (SVR) will not be abnormally low unless the arterial blood pressure (APB) is very low. A low (but not very low) arterial blood pressure (APB) coupled with a low heart rate (HR) is a normal condition which can arise during sleep, sedation, or other restful states. As a result, relying only upon the measured arterial blood pressure (APB) to determine when vasopressor intervention is indicated can result in false alarms caused by the patient entering a normal restful state during which both APB and HR decrease.

In view of the foregoing insights, a heuristic of the form: "ABP is low AND HR is slightly high or high" OR "ABP is very low" can be used to qualitatively estimate when vasopressure intervention is indicated. Such a qualitative heuristic alone is unfortunately not useful as a hemodynamic monitor or alarm. As disclosed herein, however, this heuristic can be quantified so as to provide a suitable basis for a hemodynamic monitor or alarm.

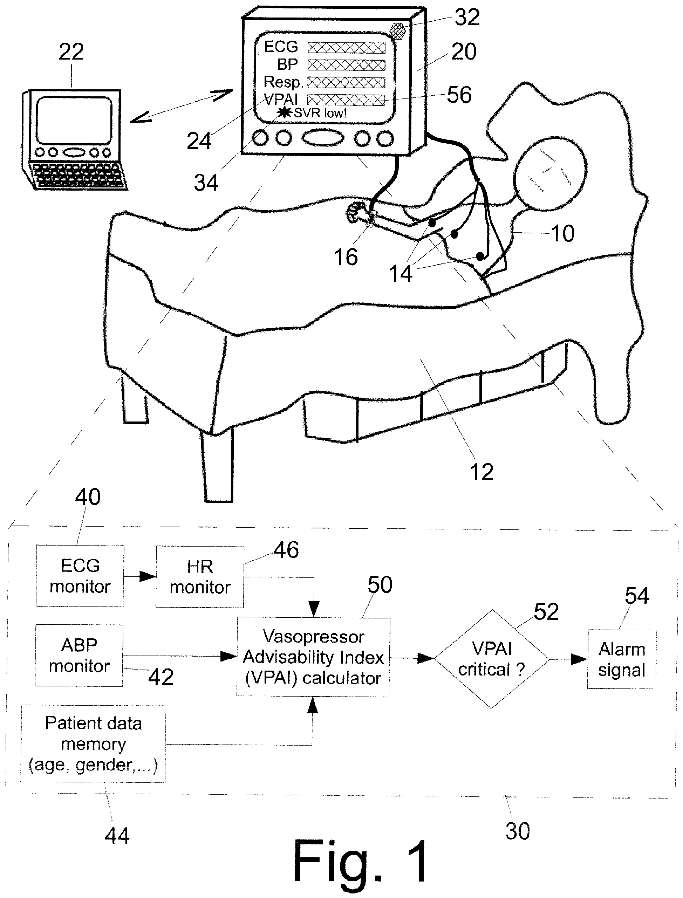

With reference to FIGURE 1, a patient 10 is shown lying in a bed 12 such as is a typical situation in a hospital, emergency room, intensive care unit (ICU), cardiac care unit (CCU), or so forth. Depending upon the patient's condition, it is also contemplated that the patient 10 may be ambulatory, residing in a wheel chair, seated in a chair, or so forth. The patient is monitored by various medical monitoring devices, including in the illustrated embodiment an electrocardiographic (ECG) instrument with ECG electrodes 14, and a blood pressure monitor 16, which may for example be a wholly non-invasive sphygmometer or a minimally invasive arterial line. The illustrated blood pressure monitor 16 is wrist-based; however, a blood pressure monitor located on the upper arm or elsewhere on the patient 10 is also contemplated. If an arterial line is used to measure blood pressure, it may optionally be incorporated into an intravenous fluid delivery line or the like. The ECG and ABP monitors 14, 16 further include associated electronics for generating and optionally performing signal processing on ECG and blood pressure signals. In the illustrated embodiment, these electronics are embodied as a unitary multi-functional patient monitor 20 that provides the electronics for both ECG and ABP monitoring, as well as optionally providing the electronics for monitoring selected other physiological parameters such as respiration rate based on suitable physiological input signals. The multi-functional patient monitor 20 is optionally locally programmable using a built-in keypad or other built-in devices (not shown), and is additionally or alternatively remotely programmable using a computer 22 or other remote device communicating with the multi-functional patient monitor 20 using a wired or wireless digital communication pathway such as a wired or wireless local area network (LAN or WLAN), bluetooth wireless connection, or so forth. The illustrated multi-functional patient monitor 20 includes a display 24 that displays measured physiological parameters such as the ECG trace, blood pressure (BP) data, respiratory data, or so forth. The display can display these parameters in various ways, such as by current numerical value, by a trace showing parameter value as a function of time, or so forth.

The multi-functional patient monitor 20, together with the ECG and blood pressure monitoring instruments, also define a hemodynamic monitor and alarm instrument. This is illustrated in FIGURE 1 by a diagrammatic processor 30 which is embodied by electronics of the multi-functional patient monitor 20. For example, the processor 30 is suitably a processor of the patient monitor 20 executing suitable software that performs processing implementing the hemodynamic instrument, displays the resulting hemodynamic parameter on the display 24, and generates an audible alarm output by an audio speaker 32, or a visual alarm 34 displayed on the display 24, or generates another perceptible alarm signal. While described with this physical construction as an illustative example, it is to be understood that the hemodynamic instrument embodiments disclosed herein can be variously physically embodied, for example as a stand-alone instrument including blood pressure and heart rate monitoring capability, or as software running on a computer or other digital device at a nurses' station, or as a pocketable or wearable portable unit, or so forth. Moreover, the hemodynamic monitoring techniques disclosed herein can also be embodied as a digital storage medium such as for example, a magnetic disk, an optical disk, an Internet server, a random access memory (RAM), a read-only memory (ROM), or so forth, that stores instructions executable by the processor 30 or by another processor to perform the hemodynamic monitoring techniques disclosed herein.

The processor 30 suitably implements ECG monitoring 40 to receive and optionally perform signal processing of the ECG signal, and further implements arterial blood pressure (ABP) moniotring 42 to receive and optionally perform signal processing of the APB signal. In the illustrated embodiment, such monitor processing 40, 42 are suitably performed by the processor 30 executing software. Alternatively, these signals may be received from elsewhere, such as from an independent ECG monitor or an independent blood pressure monitor. The processor 30 may also include or have access to a memory 44 of the patient monitor 20 storing patient information such as patient age, patient gender or sex, or so forth. Heart rate monitor processing 46 performed by the processor 30 executing suitable software extracts the heart rate as a function of time from the ECG signal.

More generally, the hemodynamic instrument receives a physiological parameter indicative of heart rate and a physiological parameter indicative of arterial blood pressure. In the illustrated embodiment, the physiological parameter indicative of heart rate is the ECG signal and the output of the heart rate monitor instrument 46 is a heart rate indicated by the physiological parameter. However, another physiological parameter indicative of heart rate could be used, such as for example the output of a fingertip $SpO_2$ monitor, and a suitable processing unit would then produce the heart rate indicated by the physiological parameter by suitable processing of the fingertip $SpO_2$ monitor signal. Similarly, in the illustrated embodiment the physiological parameter indicative of arterial blood pressure is the output of the blood pressure monitoring instrument 16, 42 which performs suitable processing of the physiological signal generated by the blood pressure monitor 16 so as to output a mean arterial blood pressure (ABP) indicated by the blood pressure monitor signal. Again, another physiological parameter indicative of mean arterial blood pressure (ABP) could be used, optionally with suitable processing to derive the ABP signal indicated by such other physiological parameter indicative of mean arterial blood pressure (ABP).

The information relevant for estimating a hemodynamic parameter correlating with systemic vascular resistance (SVR) includes: (i) the heart rate (HR) indicated by the physiological parameter indicative of heart rate; (ii) the mean arterial blood pressure (ABP) indicated by the physiological parameter indicative of arterial blood pressure; and (iii) optionally other patient data such as patient age or patient gender or sex. This information is input to a Vasopressor Advisability Index (VPAI) calculator **50,** which computes a hemodynamic parameter quantifying the heuristic "ABP is low AND HR is slightly high or high" OR "ABP is very low" where APB denotes an arterial blood pressure indicated by the physiological parameter indicative of arterial blood pressure and HR denotes a heart rate indicated by the physiological parameter indicative of heart rate.

The hemodynamic parameter computed by the VPAI calculator **50** is denoted herein as the vasopressor advisability index (VPAI), and provides a quantitative hemodynamic parameter that is (i) functionally dependent upon the quantitative heart rate (HR) measure and the quantitative mean arterial blood pressure (APB) measure and (ii) correlates with systemic vascular resistance (SVR). The VPAI is compared with a criticality criterion by a comparator **52,** and if the VPAI satisfies the criticality criterion then an alarm signal **54** is generated as a perceptible signal such as an audible alarm output by the speaker **32** or a visual alarm **34** displayed on the display **24.** Additionally or alternatively to generating the alarm signal **54,** a VPAI display **56** may be presented on the display **24** of the patient monitor **20** in the form of a plot of VPAI value versus time, or as a numerical display of the current VPAI value, or as a combination of a plot and current value numerical display, or in another suitable form. The provided VPAI information **54, 56** provides an objective and quantitative basis upon which a physician or other medical personnel can assess the advisability of administering vasopressor intervention.

Having described some embodiments of the hemodynamic monitoring instrument will illustrative reference to the illustrated embodiment of FIGURE 1, some preferred formulations of the VPAI calculation are now set forth. The function of the VPNI calculation is to quantitatively capture the pattern: "ABP is low and HR is slightly high or high" OR "ABP is very low" as an index or other quantiative value. In some preferred formulations, the VPNI is computed using fuzzy logic. In an illustrative formulation, three fuzzy variables are defined to quantitatively represent the three constituent heuristics "HR is high or slightly high", "ABP is low", and "ABP is very low".

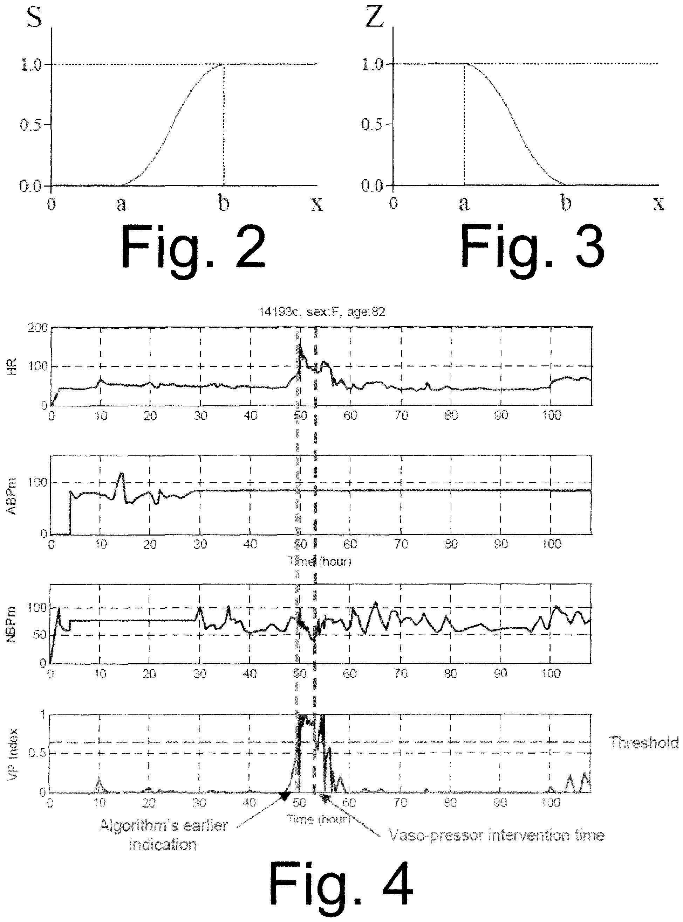

With reference to FIGURES 2 and 3, in one suitable formulation the three constituent heuristics are represented by fuzzy variables having the shape of an S-function shown in FIGURE 2, or having the shape of a Z-function shown in FIGURE 3. Quantitatively, these functions are set forth as follows:

$$S(x;a,b) = \begin{cases} 0 & , & x \leq a \\ 2\left(\dfrac{x-a}{b-a}\right)^2 & , & a < x \leq \dfrac{a+b}{2} \\ 1-2\left(\dfrac{x-b}{b-a}\right)^2 & , & \dfrac{a+b}{2} < x \leq b \\ 1 & , & b < x \end{cases} \quad (1),$$

and

$$Z(x;a,b) = 1 - S(x;a,b) \quad (2).$$

Using these functions, the heuristic "HR is slightly high or high" is quantified as:

$$\mu_{HR\_is\_slightlyHigh\_or\_high} = S(HR \; ; \; 70+AF_{HR}+SF_{HR} \; , \; 120+AF_{HR}+SF_{HR}) \quad (3),$$

where HR is the heart rate in beats per minute indicated by the physiological parameter indicative of heart rate, $AF_{HR}$ is an optional patient age adjustment factor, $SF_{HR}$ is an optional patient gender or sex adjustment factor, the S-function low-end boundary $a$ is $70+AF_{HR}+SF_{HR}$, and the S-function high-end boundary b is $120+AF_{HR}+SF_{HR}$.

In similar fashion, quantitative fuzzy variables may be defined for the remaining constituent heuristics as follows:

$$\mu_{APB\_is\_low} = Z(ABP \; ; \; 60+AF1_{ABP}+SF1_{ABP} \; , \; 80+AF1_{ABP}+SF1_{ABP}) \quad (4),$$

and

$$\mu_{APB\_is\_veryLow} = Z(ABP \; ; \; 40+AF2_{ABP}+SF2_{ABP} \; , \; 60+AF2_{ABP}+SF2_{ABP}) \qquad (5),$$

where ABP is the mean arterial blood pressure in mmHg indicated by the physiological parameter indicative of arterial blood pressure, and $AF1_{ABP}$ and $AF2_{ABP}$ are optional patient age adjustment factors, and $SF1_{ABP}$ and $SF2_{ABP}$ are optional patient gender or sex adjustment factors.

The optional adjustment factors $AF_{HR}$, $AF1_{ABP}$, $AF2_{ABP}$, $SF_{HR}$, $SF1_{ABP}$, and $SF2_{ABP}$ are suitably derived from patient data compilations representing typical heart rates and mean arterial blood pressure values as a function of patient age and patient gender or sex. For example, it is known that the aterial blood pressure tends to increase monotonically with patient age, so the optional adjustment factor $AF1_{ABP}$ is suitably a monotonically increasing function of patient age reflecting this known trend. It is contemplateed for some of these adjustment factors to have negative values. Adjustment factors for patient age and patient gender or sex are expressly set forth herein as illustrative examples. However, it is contemplated that adjustment factors for other patient characteristics may be incorporated. Additionally or alternatively, it is contemplated to have patient-specific boundaries for the fuzzy variables, for example based on actually recorded heart rate values provided in the medical history of a specific patient.

The quantitative fuzzy variable set forth in each of Equations (3), (4), and (5) have output values in the range [0,1] due to the limits set by the S- and Z-functions. For $\mu_{HR\_is\_slightlyHigh\_or\_high}$ higher values indicate higher agreement with the heuristic "Heart rate (HR) is slightly high or high". For $\mu_{APB\_is\_low}$, higher values indicate higher agreement with the heuristic "Mean arterial blood pressure (APB) is low". For $\mu_{APB\_is\_veryLow}$, higher values indicate higher agreement with the heuristic "Mean arterial blood pressure (APB) is very low". The vasopressor advisability index, denoted $\mu_{VRAI}$, is suitably defined as a fuzzy composite variable according to:

$$\mu_{VPAI} = (\mu_{APB\_is\_low} \wedge \mu_{HR\_is\_slightlyHigh\_or\_high}) \vee \mu_{APB\_is\_veryLow} \qquad (6),$$

where the symbol "$\wedge$" denotes the standard fuzzy intersection, defined as $\mu_A(x) \wedge \mu_B(x) = min\{\mu A(x), \mu_B(x)\}$, and the symbol "v" denotes the standard fuzzy union, defined as $\mu_A(x) \vee \mu_B(x) = max\{\mu_A(x), \mu_B(x)\}$. The vasopressor advisability index ($\mu_{VPAI}$) is also bounded to lie in the range [0,1], with higher values indicating higher agreement with the heuristic "Vasopressor intervention is advisable".

The criticality criterion implemented by the comparator **52** can be constructed in various ways. One suitable criterion is:

$$\mathbf{IF} \; (\mu_{VPAI} > I_{th}) \; \mathbf{THEN} \; \textit{Vasopressor intervention advised} \qquad (7)$$

where $I_{th}$ is a threshold value. The criticality criterion can also be expressed using a fuzzy conditional statement according to:

$$\mathbf{IF} \; (\mu_{APB\_is\_low} \; \mathbf{AND} \; \mu_{HR\_is\_slightlyHigh\_or\_high}) \; \mathbf{OR} \; \mu_{APB\_is\_veryLow}$$

$$\mathbf{THEN} \; \textit{Vasopressor intervention advised} \qquad (8).$$

In Equation (8), the first part ($\mu_{APB\_is\_low}$ **AND** $\mu_{HR\_is\_slightlyHigh\_or\_high}$) identifies abnormal events when the blood pressure drops noticeably while the heart rate is faster than usual. The second part $\mu_{APB\_is\_veryLow}$ identifies the situation in which the blood pressure decreases to a critically low value, regardless of the heart rate. The threshold for activating the alarm (e.g., the threshold $I_{th}$ of Equation (7)) is suitably obtained empirically by analysis of reference patient databases, or may also be determined by clinical users according to specific patient situations. In some embodiments, the hemodynamic monitoring instrument has a user-selectable threshold that is adjustable.

To summarize, the hemodynamic monitoring instrument operates in the following way. For each time point, the instrument receives a heart rate (HR) value indicated by the physiological parameter indicative of heart rate, a mean arterial blood pressure (APB) value indicated by the physiological parameter indicative of arterial blood pressure, and optionally receives further relevant information such as patient age, patient gender or sex, or so forth, and generates a vasopressor advisability index ($\mu_{VPAI}$) according to Equation (6). The vasopressor advisability index ($\mu_{VPAI}$) is displayed in real-time, optionally along with the heart rate HR and ABP values, and is compared to a threshold or other criticality criterion. If the vasopressor advisability index ($\mu_{VPAI}$) satisfies the criticality criterion, then a vasopressor intervention advisability alarm is issued which indicates advisability of vaso-pressor intervention or another remediation of the abnormal systemic vascular resistance (SVR) condition.

In the following, some illustrative examples of operation of a hemodynamic monitoring instrument constructed in substantial accordance with the teachings set forth herein are set forth.

With reference to FIGURE 4, an ICU record is shown for an 82-year old female patient in the MIMIC-II database. *See* Saeed et al., "MIMIC-II: A massive temporal ICU patient database to support intelligent patient monitoring", Computers in Cardiology 2002; 29:641-44. The panels of FIGURE 4 plot the patient heart rate (HR) measurements, patient mean arterial blood pressure (ABPm) measurements, and mean non-invasive blood pressure (NBPm) measurements. Note that the flat-line periods in the ABPm and NBPm traces refer to time intervals during which the measurements were not taken or were not available in the MIMIC-II database. The bottom panel plots the vasopressor advisability index ($\mu$VPAI, denoted "VP Index" in the relevant panel of FIGURE 4) computed for those time intervals during which either APBm or NBPm values were available. In the bottom panel, the threshold $I_{th}$ of Equation (7) is indicated by a horizontal dashed line.

As seen in FIGURE 4, the patient's vasopressor advisability index ($\mu$VPAI) value became high (exceeding a preset threshold of about 0.6) around at 50 hours, this point in time being indicated by the lefthand vertical dashed line in FIGURE 4, indicating that there was a critical event appearing corresponding to a low systemic vascular resistance (SVR). However, the patient's medication record stored in the MIMIC-II Database shows that an actual vasopressor (Neosynephrine) intervention was applied to the patient at around 53 hours, this point in time being indicated by the righthand vertical dashed line in FIGURE 4. This is about 2.5 hours after the vasopressor advisability index ($\mu_{VPAI}$) advised to initiate vasopressor intervention. The vasopressor intervention was successful, as indicated by a substantial decrease in the vasopressor advisability index ($\mu_{VPAI}$) shortly after initiation of the vasopressor intervention.

As further seen in FIGURE 4, the NBPm also dropped to a noticeable low level at times other than around the 50 hour interval indicated by the vasopressor advisability index ($\mu_{VPAI}$) as corresponding to an event calling for vasopressor intervention. Such noticeable decreases in the NBPm occurred, for example, in the 38-48 hour interval, in the 60-70 hour interval, and in the 80-90 hour interval. However, the vasopressor advisability index ($\mu_{VPAI}$) did not produce high values in those periods, indicating that no false alarms were triggered by these blood pressure decreases. In contrast, a physician monitoring the blood pressure without having the benefit of the vasopressor advisability index ($\mu_{VPAI}$) might have elected to initiate (unnecessary) vasopressor intervention at one or more of these time intervals.

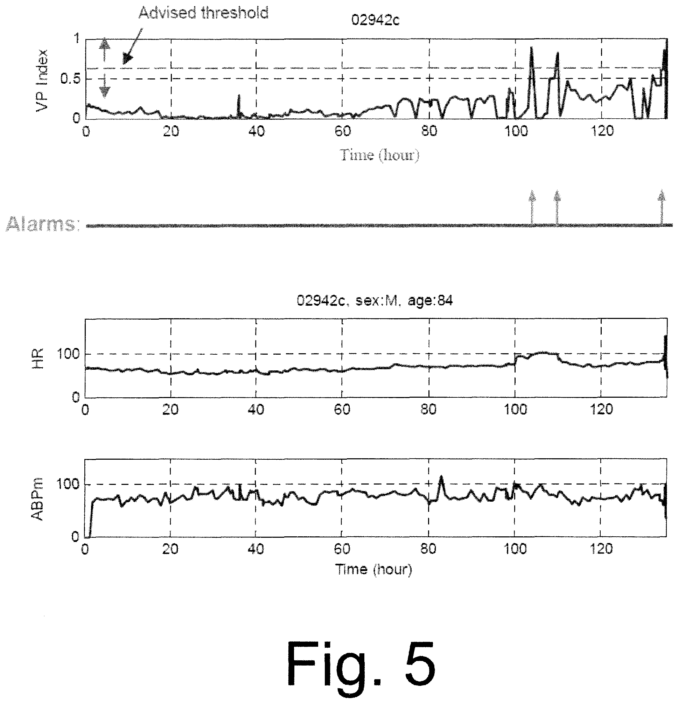

With reference to FIGURE 5, another illustrative example is shown, in which the vasopressor advisability index ($\mu_{VPAI}$) is computed for patient monitoring data of an 84 year old male patient, again taken from the MIMIC-II database. The vasopressor advisability index ($\mu_{VPAI}$) values are computed as a function of time with adjustments for the patient's age and gender, using the heart rate (HR) and mean arterial blood pressure (ABP) measurements stored in the MIMIC-II database. The vasopressor advisability index ($\mu_{VPAI}$, denoted in FIGURE 5 as "VP Index") is plotted in the top panel of FIGURE 5. The threshold $I_{th}$ of Equation (7) is indicated by a horizontal dashed line in FIGURE 5. When the vasopressor advisability index exceeds the threshold $I_{th}$, a critical event is detected and a perceptible alarm is generated to notify the physician or other medical personnel that vasopressor intervention is deemed to be advisable. Upon perceiving the alarm, the physician or other medical personnel can review the current and recent physiological data (for example using the patient monitor **20** of FIGURE 1) to confirm the occurrence of an abnormal hemodynamic event indicative of a low systemic vascular resistance. This confirmation may entail, for example, consideration of the heart rate (HR) and mean arterial blood pressure (ABPm) readings shown in FIGURE 5 and suitably plotted along with the vasopressor advisability index on the display **24** of the patient monitor **20.**

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

**Claims**

1. A hemodynamic monitoring instrument comprising:

   a processor (30) arranged to receive a physiological parameter indicative of heart rate and a physiological parameter indicative of arterial blood pressure and configured to compute (50) a hemodynamic parameter correlating with systemic vascular resistance (SVR) based on the received physiological parameter indicative of heart rate and the received physiological parameter indicative of arterial blood pressure; and
   an output device including least one of (i) a display (24) configured to display the computed hemodynamic parameter and (ii) an alarm (32, 34) configured to generate a perceptible signal responsive to the computed hemodynamic parameter satisfying an alarm criterion (52),
**characterized in that** the processor (30) is configured to compute (50) the hemodynamic parameter correlating with SVR based on:

a first fuzzy membership function indicative of whether a heart rate indicated by the physiological parameter indicative of heart rate is slightly high or high;
a second fuzzy membership function indicative of whether an arterial blood pressure indicated by the physiological parameter indicative of arterial blood pressure is low; and
a third fuzzy membership function indicative of whether the arterial blood pressure indicated by the physiological parameter indicative of arterial blood pressure is very low,
wherein the processor (30) is further configured to compute (50) the hemodynamic parameter correlating with SVR based on:

a first term defined by conjunctive combination of the first fuzzy membership function and the second fuzzy membership function, and
a second term corresponding to the third fuzzy membership function,
the hemodynamic parameter quantifying the heuristic "ABP is low AND HR is slightly high or high" OR "ABP is very low" where APB denotes an arterial blood pressure indicated by the physiological parameter indicative of arterial blood pressure and HR denotes a heart rate indicated by the physiological parameter indicative of heart rate.

2. The hemodynamic monitoring instrument as set forth in claim 1, wherein the instrument is integral with a patient monitor (20) configured to display at least one of (i) a heart rate derived from the physiological parameter indicative of heart rate and (ii) an arterial blood pressure derived from the physiological parameter indicative of arterial blood pressure.

3. The hemodynamic monitoring instrument as set forth in claim 1, wherein the processor (30) is configured to increase HR by $AF_{HR}$ where $AF_{HR}$ denotes a correction term based on patient age.

4. The hemodynamic monitoring instrument as set forth in claim 1, wherein the processor (30) is configured to increase ABP by $AF_{ABP}$ where $AF_{ABP}$ denotes a correction term based on patient age that is monotonically increasing with patient age.

5. The hemodynamic monitoring instrument as set forth in claim 1, wherein the output device comprises:

a display (24) configured to plot the computed hemodynamic parameter as a function of time.

6. A hemodynamic monitoring method comprising:

computing a quantitative hemodynamic parameter that is (i) functionally dependent upon a quantitative heart rate (HR) measure and a quantitative arterial blood pressure (ABP) measure and (ii) correlates with systemic vascular resistance (SVR) and (iii) quantifies the heuristic "quantitative ABP measure is low AND quantitative HR measure is slightly high or high" OR " quantitative ABP measure is very low", wherein the computing of the quantitative hemodynamic parameter comprises:

computing a first fuzzy variable indicative of whether the quantitative HR measure is slightly high or high;
computing a second fuzzy variable indicative of whether the quantitative ABP measure is low;
computing a third fuzzy variable indicative of whether the quantitative ABP measure is very low;
computing a fuzzy composite variable combining the first, second, and third fuzzy variables using fuzzy combinational operators selected from the group consisting of fuzzy intersection and fuzzy union, the fuzzy composite variable correlating with systemic vascular resistance (SVR); and
at least one of (i) displaying the quantitative hemodynamic parameter and (ii) generating a perceptible signal indicative of an abnormal hemodynamic condition conditional upon the computed hemodynamic parameter satisfying an alarm criterion.

7. The hemodynamic monitoring method as set forth in claim 6, wherein computing of the quantitative hemodynamic parameter further comprises increasing the quantitative HR measure by $AF_{HR}$ where $AF_{HR}$ denotes a correction term based on patient age.

**8.** The hemodynamic monitoring method as set forth in claim 6, wherein computing of the quantitative hemodynamic parameter further comprises increasing the quantitative ABP measure by $AF_{ABP}$ where $AF_{ABP}$ denotes a correction term based on patient age that is monotonically increasing with patient age.

**9.** The hemodynamic monitoring method as set forth in claim 6, wherein computing of the quantitative hemodynamic parameter comprises:

computing a quantitative hemodynamic parameter having a value substantially equivalent to the result $\mu_{VPAI}$ of the equation:

$$\mu_{VPAI} = \left( \mu_{APB\_is\_low} \wedge \mu_{HR\_is\_slightlyHigh\_or\_high} \right) \vee \mu_{APB\_is\_veryLow}$$

where:

$$\mu_{HR\_is\_slightlyHigh\_or\_high} = S(HR \; ; \; 70+F_{HR} \; , \; 120+F_{HR})$$

and

$$\mu_{APB\_is\_low} = Z(ABP \; ; \; 60+F1_{ABP} \; , \; 80+F1_{ABP})$$

and

$$\mu_{APB\_is\_veryLow} = Z(ABP \; ; \; 40+F2_{ABP} \; , \; 60+F2_{ABP})$$

and where HR denotes the quantitative HR measure, ABP denotes the quantitative ABP measure, and $F_{HR}$, $F1_{ABP}$, and $F2_{ABP}$ denote patient-dependent adjustment factors.

**10.** A computer medium storing instructions executable to control a computer (20) and display (24) to perform the method of claim 6.

**11.** A hemodynamic monitoring device including a display (24) and a processor (30) programmed to perform the method of claim 6.

**Patentansprüche**

**1.** Hämodynamisches Überwachungsinstrument, das Folgendes umfasst:

einen Prozessor (30), der davor vorgesehen ist, einen die Herzfrequenz angebenden physiologischen Parameter und einen den arteriellen Blutdruck angebenden physiologischen Parameter zu empfangen, und der konfiguriert ist, um einen mit dem systemischen Gefäßwiderstand (engl. systemic vascular resistance, SVR) korrelierenden hämodynamischen Parameter basierend auf dem empfangenen die Herzfrequenz angebenden physiologischen Parameter und dem empfangenen den arteriellen Blutdruck angebenden physiologischen Parameter zu berechnen (50); und
eine Ausgabevorrichtung umfassend mindestens entweder (i) eine Anzeige (24), die konfiguriert ist, um den berechneten hämodynamischen Parameter anzuzeigen, oder (ii) einen Alarm (32, 34), der konfiguriert ist, um ein wahrnehmbares Signal in Reaktion darauf zu erzeugen, dass der berechnete hämodynamische Parameter ein Alarmkriterium (52) erfüllt,
**dadurch gekennzeichnet, dass** der Prozessor (30) konfiguriert ist, um den mit dem systemischen Gefäßwiderstand korrelierenden hämodynamischen Parameter basierend auf Folgendem zu berechnen (50):

einer ersten Fuzzy-Zugehörigkeitsfunktion (engl. fuzzy membership function), die angibt, ob eine durch den die Herzfrequenz angebenden physiologischen Parameter angegebene Herzfrequenz etwas erhöht oder hoch ist;

einer zweiten Fuzzy-Zugehörigkeitsfunktion, die angibt, ob ein durch den den arteriellen Blutdruck angebenden physiologischen Parameter angegebener arteriellen Blutdruck niedrig ist; und

einer dritten Fuzzy-Zugehörigkeitsfunktion, die angibt, ob der durch den den arteriellen Blutdruck angebenden physiologischen Parameter angegebene arterielle Blutdruck sehr niedrig ist,

wobei der Prozessor (30) weiterhin konfiguriert ist, um den mit SVR korrelierenden hämodynamischen Parameter basierend auf Folgendem zu berechnen (50):

einem ersten Term, der durch konjunktive Kombination der ersten Fuzzy-Zugehörigkeitsfunktion und der zweiten Fuzzy-Zugehörigkeitsfunktion definiert ist, und

einen zweiten Term, der der dritten Fuzzy-Zugehörigkeitsfunktion entspricht,

wobei der hämodynamische Parameter die Heuristik "ABP ist niedrig UND die HR ist etwas erhöht oder hoch" ODER "ABP ist sehr niedrig" quantifiziert, wobei APB einen arteriellen Blutdruck bezeichnet, der durch den den arteriellen Blutdruck angebenden physiologischen Parameter angegeben wird, und HR eine Herzfrequenz bezeichnet, die durch den die Herzfrequenz angebenden physiologischen Parameter angegeben wird.

2. Hämodynamisches Überwachungsinstrument nach Anspruch 1, wobei das Instrument mit einem Patientenmonitor (20) integriert ist, der konfiguriert ist, um mindestens entweder (i) eine von dem die Herzfrequenz angebenden physiologischen Parameter abgeleitete Herzfrequenz anzuzeigen oder (ii) einen von dem den arteriellen Blutdruck angebenden physiologischen Parameter abgeleiteten arteriellen Blutdruck anzuzeigen.

3. Hämodynamisches Überwachungsinstrument nach Anspruch 1, wobei der Prozessor (30) konfiguriert ist, um HR um $AF_{HR}$ zu erhöhen, wobei $AF_{HR}$ einen auf dem Alter des Patienten basierenden Korrekturterm bezeichnet.

4. Hämodynamisches Überwachungsinstrument nach Anspruch 1, wobei der Prozessor (30) konfiguriert ist, um ABP um $AF_{ABP}$ zu erhöhen, wobei $AF_{ABP}$ einen auf dem Alter des Patienten basierenden Korrekturterm bezeichnet, der mit dem Alter des Patienten monoton ansteigt.

5. Hämodynamisches Überwachungsinstrument nach Anspruch 1, wobei die Ausgabevorrichtung Folgendes umfasst:

eine Anzeige (24), die konfiguriert ist, um den berechneten hämodynamischen Parameter als eine Funktion der Zeit grafisch darzustellen.

6. Hämodynamisches Überwachungsverfahren, das Folgendes umfasst:

Berechnen eines quantitativen hämodynamischen Parameters, der (i) funktionell von einem quantitativen Herzfrequenz- (HR) Wert und einem quantitativen arteriellen Blutdruck- (ABP) Wert abhängt und (ii) mit dem systemischen Gefäßwiderstand (SVR) korreliert und (iii) die Heuristik "quantitativer ABP-Wert ist niedrig UND quantitativer HR-Wert ist etwas erhöht oder hoch" ODER "quantitativer ABP-Wert ist niedrig" quantifiziert, wobei das Berechnen des quantitativen hämodynamischen Parameters Folgendes umfasst:

Berechnen einer ersten Fuzzy-Variablen, die angibt, ob der quantitative HR-Wert etwas erhöht oder hoch ist;

Berechnen einer zweiten Fuzzy-Variablen, die angibt, ob der quantitative ABP-Wert niedrig ist;

Berechnen einer dritten Fuzzy-Variablen, die angibt, ob der quantitative ABP-Wert sehr niedrig ist;

Berechnen einer zusammengesetzten Fuzzy-Variablen, die die erste, die zweite und die dritte Fuzzy-Variable unter Verwendung von kombinatorischen Fuzzy-Operatoren ausgewählt aus der Gruppe bestehend aus Fuzzy-Schnittmenge und Fuzzy-Vereinigung kombiniert, wobei die zusammengesetzte Fuzzy-Variable mit dem systemischen Gefäßwiderstand (SVR) korreliert; und

mindestens entweder (i) Anzeigen des quantitativen hämodynamischen Parameters oder (ii) Erzeugen eines wahrnehmbaren Signals, das einen abnormalen hämodynamischen Zustand angibt, sofern der berechnete hämodynamische Parameter ein Alarmkriterium erfüllt.

7. Hämodynamisches Überwachungsverfahren nach Anspruch 6, wobei das Berechnen des quantitativen hämodynamischen Parameters weiterhin das Erhöhen des quantitativen HR-Werts um $AF_{HR}$ umfasst, wobei $AF_{HR}$ einen auf dem Alter des Patienten basierenden Korrekturterm bezeichnet.

8. Hämodynamisches Überwachungsverfahren nach Anspruch 6, wobei das Berechnen des quantitativen hämodynamischen Parameters weiterhin das Erhöhen des quantitativen ABP-Werts um $AF_{ABP}$ umfasst, wobei $AF_{ABP}$ einen auf dem Alter des Patienten basierenden Korrekturterm bezeichnet, der mit dem Alter des Patienten monoton ansteigt.

9. Hämodynamisches Überwachungsverfahren nach Anspruch 6, wobei das Berechnen des quantitativen hämodynamischen Parameters Folgendes umfasst:

Berechnen eines quantitativen hämodynamischen Parameters mit einem Wert, der im Wesentlichen dem Ergebnis $\mu_{VPAI}$ der folgenden Gleichung entspricht:

$$\mu_{VPAI} = \left(\mu_{APB\_ist\_niedrig} \wedge \mu_{HR\_ist\_etwasErhöht\_oder\_hoch}\right) \vee \mu_{APB\_ist\_sehrNiedrig}$$

wobei:

$$\mu_{HR\_ist\_etwasErhöht\_oder\_hoch} = S\left(HR; 70+F_{HR}, 120+F_{HR}\right)$$

und

$$\mu_{APB\_ist\_niedrig} = Z\left(ABP; 60+F1_{ABP}, 80+F1_{ABP}\right)$$

und

$$\mu_{APB\_ist\_sehrNiedrig} = Z\left(ABP; 40+F2_{ABP}, 60+F2_{ABP}\right)$$

und wobei HR den quantitativen HR-Wert bezeichnet, ABP den quantitativen ABP-Wert bezeichnet und $F_{HR}$, $F1_{ABP}$ und $F2_{ABP}$ patientenabhängige Anpassungsfaktoren bezeichnen.

10. Computermedium mit darauf gespeicherten Anweisungen, die ausführbar sind, um einen Computer (20) und eine Anzeige (24) zu steuern, um das Verfahren nach Anspruch 6 durchzuführen.

11. Hämodynamische Überwachungsvorrichtung umfassend eine Anzeige (24) und einen Prozessor (30), der programmiert ist, um das Verfahren nach Anspruch 6 durchzuführen.

**Revendications**

1. Instrument de surveillance hémodynamique comprenant :

un processeur (30) agencé pour recevoir un paramètre physiologique indicatif d'un pouls et un paramètre physiologique indicatif d'une tension artérielle et configuré pour calculer (50) un paramètre hémodynamique en corrélation avec une résistance vasculaire systémique (SVR) sur la base du paramètre physiologique reçu indicatif d'un pouls et du paramètre physiologique reçu indicatif d'une tension artérielle ; et
un dispositif de sortie comprenant l'un de (i) un affichage (24) configuré pour afficher le paramètre hémodynamique calculé et (ii) une alarme (32, 34) configurée pour générer un signal perceptible en réponse au paramètre hémodynamique calculé satisfaisant un critère d'alarme (52),
**caractérisé en ce que** le processeur (30) est configuré pour calculer (50) le paramètre hémodynamique en corrélation avec une SVR sur la base de :

une première fonction d'abonnement floue indiquant si un pouls indiqué par le paramètre physiologique indicatif d'un pouls est légèrement élevé ou est élevé ;
une deuxième fonction d'abonnement floue indiquant si une tension artérielle indiquée par le paramètre physiologique indicatif d'une tension artérielle est basse ; et
une troisième fonction d'abonnement floue indiquant si la tension artérielle indiquée par le paramètre physiologique indicatif d'une tension artérielle est très basse,

dans lequel le processeur (30) est en outre configuré pour calculer (50) le paramètre hémodynamique en corrélation avec une SVR sur la base de :

> un premier terme défini par une combinaison conjonctive de la première fonction d'abonnement floue et de la deuxième fonction d'abonnement floue, et
> un deuxième terme correspondant à la troisième fonction d'abonnement floue,
> le paramètre hémodynamique quantifiant « ABP est basse ET HR est légèrement élevé ou est élevé » OU « ABP est très basse » heuristique, où ABP représente une tension artérielle indiquée par le paramètre physiologique indicatif d'une tension artérielle et HR représente un pouls indiqué par le paramètre physiologique indicatif d'un pouls.

2. Instrument de surveillance hémodynamique selon la revendication 1, dans lequel l'instrument fait partie intégrante d'un moniteur de patient (20) configuré pour afficher au moins l'un de (i) un pouls dérivé du paramètre physiologique indicatif d'un pouls et (ii) une tension artérielle dérivée du paramètre physiologique indicatif d'une tension artérielle.

3. Instrument de surveillance hémodynamique selon la revendication 1, dans lequel le processeur (30) est configuré pour accroître HR de $AF_{HR}$, où $AF_{HR}$ représente un terme de correction basé sur un âge de patient.

4. Instrument de surveillance hémodynamique selon la revendication 1, dans lequel le processeur (30) est configuré pour accroître ABP de $AF_{ABP}$, où $AF_{ABP}$ représente un terme de correction basé sur un âge de patient augmentant de manière monotone avec un âge de patient.

5. Instrument de surveillance hémodynamique selon la revendication 1, dans lequel le dispositif de sortie comprend :

un affichage (24) configuré pour tracer le paramètre hémodynamique calculé en fonction du temps.

6. Procédé de surveillance hémodynamique comprenant :

le calcul d'un paramètre hémodynamique quantitatif qui (i) dépend fonctionnellement d'une mesure de pouls (HR) quantitative et d'une mesure de tension artérielle (ABP) quantitative et (ii) est en corrélation avec une résistance vasculaire systémique (SVR) et (iii) quantifie « la mesure ABP quantitative est basse ET la mesure HR quantitative est légèrement élevée ou est élevée » OU « la mesure ABP quantitative est très basse » heuristique, dans lequel le calcul du paramètre hémodynamique quantitatif comprend :

le calcul d'une première variable floue indiquant si la mesure HR quantitative est légèrement élevée ou est élevée ;
le calcul d'une deuxième variable floue indiquant si la mesure ABP quantitative est basse ; et
le calcul d'une troisième variable floue indiquant si la mesure ABP quantitative est très basse ;
le calcul d'une variable composite floue combinant les première, deuxième et troisième variables floues en utilisant des opérateurs combinatoires flous sélectionnés dans le groupe se composant d'une intersection floue et d'une union floue, la variable composite floue étant en corrélation avec une résistance vasculaire systémique (SVR) ; et
au moins l'un de (i) l'affichage du paramètre hémodynamique quantitatif et (ii) la génération d'un signal perceptible indicatif d'un état hémodynamique anormal en réponse au paramètre hémodynamique calculé satisfaisant un critère d'alarme.

7. Procédé de surveillance hémodynamique selon la revendication 6, dans lequel le calcul du paramètre hémodynamique quantitatif comprend en outre l'accroissement de la mesure HR quantitative de $AF_{HR}$, où $AF_{HR}$ représente un terme de correction basé sur un âge de patient.

8. Procédé de surveillance hémodynamique selon la revendication 6, dans lequel le calcul du paramètre hémodynamique quantitatif comprend en outre l'accroissement de la mesure ABP quantitative de $AF_{ABP}$, où $AF_{ABP}$ représente un terme de correction basé sur un âge de patient augmentant de manière monotone avec un âge de patient.

9. Procédé de surveillance hémodynamique selon la revendication 6, dans lequel le calcul du paramètre hémodynamique quantitatif comprend :

le calcul d'un paramètre hémodynamique quantitatif ayant une valeur sensiblement équivalente au résultat $\mu_{VPAI}$ de l'équation :

$$\mu_{VPAI} = \left(\mu_{APB\_is\_low} \wedge \mu_{HR\_is\_slightlyHigh\_or\_high}\right) \vee \mu_{APB\_is\_veryLow}$$

où

$$\mu_{HR\_is\_slightlyHigh\_or\_high} = S(HR ; 70+F_{HR} , 120+F_{HR})$$

et

$$\mu_{APB\_is\_low} = Z(ABP ; 60+F1_{ABP} , 80+F1_{ABP})$$

et

$$\mu_{APB\_is\_veryLow} = Z(ABP ; 40+F2_{ABP} , 60+F2_{ABP})$$

et où HR représente la mesure HR quantitative, ABP représente la mesure ABP quantitative, et $F_{HR}$, $F1_{ABP}$ et $F2_{ABP}$ représentent des facteurs d'ajustement en fonction du patient.

10. Support informatique mémorisant des instructions exécutables pour commander à un ordinateur (20) et à un affichage (24) d'effectuer le procédé selon la revendication 6.

11. Dispositif de surveillance hémodynamique comprenant un affichage (24) et un processeur (30) programmés pour effectuer le procédé selon la revendication 6.

**Fig. 1**

# Fig. 2

# Fig. 3

14193c, sex:F, age:82

Threshold

Algorithm's earlier indication

Vaso-pressor intervention time

# Fig. 4

# Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007060559 A2

### Non-patent literature cited in the description

- **BECKER K et al.** Fuzzy logic approaches to intelligent alarms. *IEEE ENGINEERING IN MEDICINE AND BIOLOGY MAGAZINE 1994 NOV-DEC IEEE US,* November 1994, vol. 13 (5), 710-716

- **SAEED et al.** MIMIC-II: A massive temporal ICU patient database to support intelligent patient monitoring. *Computers in Cardiology,* 2002, vol. 29, 641-44